# EUROPEAN PATENT APPLICATION

(11) **EP 4 420 667 A1**
(43) Date of publication of application: **28.08.2024**
(21) Application number: 22884041.9
(22) Date of filing: 19.10.2022
(51) Int. Cl.: A61K 35/744, A61P 25/28, A61P 25/16, A23L 33/135, A23K 10/16, C12N 1/20, C12R 1/00

(54) **NOVEL STRAIN HAVING ANTAGONISM AGAINST PROTEUS MIRABILIS AND EXCELLENT EFFECT ON NEURODEGENERATIVE DISEASE, AND USE THEREOF**

(30) Priority: 19.10.2021 KR 20210139579
(71) Applicant: Metacen Therapeutics Co., Ltd, Suwon-si, Gyeonggi-do 16229 (KR)
(72) Inventor: OH, Myung Sook, Seoul 06291 (KR); HUH, Eugene, Seoul 04202 (KR); PARK, Myoung Gyu, Yongin-si Gyeonggi-do 17103 (KR); JEH, Hoon Sung, Daejeon 34198 (KR)
(74) Representative: Hoffmann Eitle
(86) International application number: PCT/KR2022/015983
(87) International publication number: WO 2023/068819

(57) **Abstract**

The present invention relates to a novel *Weissella cibaria* strain and a use thereof. The novel *Weissella cibaria* strain according to the present invention and a composition comprising the same have not only antimicrobial activity against *Proteus mirabilis,* but also protective ability against neurotoxicity-induced apoptosis of neurons, inhibition ability against aggregation of α-synuclein, and safety, and thus can be utilized as a pharmaceutical composition, a food composition, and an animal feed composition, which are excellent in prevention, amelioration, or treatment of neurodegenerative diseases.

## Description

### Technical Field

The present invention relates to a novel *Weissella cibaria* strain and a use thereof. Specifically, the present invention relates to a novel *Weissella cibaria* strain having antimicrobial activity against *Proteus mirabilis* and excellent effect on a neurodegenerative disease, and to a composition for the prevention, amelioration, or treatment of a neurodegenerative disease comprising the same.

### Background Art

Recently, with the rapid increase in the elderly population and the increase in the number of patients with various neurodegenerative diseases, interest in the treatment and prevention thereof is increasing. Neurodegenerative disease is a disease that causes various conditions such as motor disorder, memory disorder, and cognitive disorder due to a decrease or loss of nerve cell function. Nerve cells die in large numbers every day not only in neurological diseases but also in normal adult brains, and the number of nerve cells that die increases exponentially with aging.

Major diseases belonging to neurodegenerative diseases include Alzheimer's disease, Parkinson's disease, Lou Gehrig's disease, Huntington's disease, and the like, and the pathogenesis of these diseases has not been fully revealed to date. Acetylcholinesterase inhibitors or NMDA (N-methyl-D-aspartate) receptor antagonists are used as therapeutic agents for Alzheimer's disease, and L-dopa, dopamine agonists, MAO-B inhibitors, or COMT inhibitors are used as therapeutic agents for Parkinson's disease, and dopamine D2 receptors and the like are used as therapeutic agents for Huntington's disease. However, since the above therapeutic agents all target the neurotransmission process, methods using the above therapeutic agents are merely alleviating symptoms, not fundamental treatment. Therefore, there is a continuous need for novel drugs capable of fundamental treatment.

Meanwhile, the brain and intestines are closely connected and interact with each other, and intestinal microorganisms present in the intestines are known to maintain homeostasis in the intestinal environment and are also involved in the production of neurotransmitters in the brain. In particular, it has been revealed that changes in intestinal microorganisms are associated with not only metabolic diseases such as obesity and diabetes mellitus, but also mental diseases such as depression and autism, and neurodegenerative diseases such as Alzheimer's disease and Parkinson's disease.

In addition, there are clinical reports on the association between the increase in *Enterobacteriaceae* family strains in the stool of Parkinson's patients and Parkinson's disease. In particular, Korean Patent Registration No. 10-1860566 discloses that a *Proteus mirabilis* strain belonging to the *Enterobacteriaceae* family exists in an animal model of Parkinson's disease, and that the *Proteus mirabilis* strain is directly involved in causing Parkinson's disease.

Accordingly, the present inventors discovered a *Weissella cibaria* strain that inhibits the growth of a *Proteus mirabilis* strain and confirmed excellent effect of the above strain on ameliorating a neurodegenerative disease. Based on the above, the present inventors completed the present invention.

### Prior Art Document

### Patent Document

(Patent Document 1) Korean Patent Registration No. 10-1860566

### Detailed Description of Invention

### Technical Problem

An object of the present invention is to provide a *Weissella cibaria* strain having antimicrobial activity against *Proteus mirabilis,* protective ability against apoptosis of neurons, and inhibition ability against aggregation of α-synuclein.

Another object of the present invention is to provide a pharmaceutical composition for preventing or treating a neurodegenerative disease, comprising the *Weissella cibaria* strain.

Another object of the present invention is to provide a food composition for preventing or ameliorating a neurodegenerative disease, comprising the *Weissella cibaria* strain.

Another object of the present invention is to provide an animal feed composition for preventing or ameliorating a neurodegenerative disease, comprising the *Weissella cibaria* strain.

### Solution to Problem

The present invention provides a pharmaceutical composition for preventing or treating a neurodegenerative disease, comprising a *Weissella cibaria* strain.

The present invention also provides a food composition for preventing or ameliorating a neurodegenerative disease, comprising a *Weissella cibaria* strain.

The present invention also provides an animal feed composition for preventing or ameliorating a neurodegenerative disease, comprising a *Weissella cibaria* strain.

The *Weissella cibaria* strain of the present invention includes *Weissella cibaria* CHK903, *Weissella cibaria* SGW054, *Weissella cibaria* SPW2014, *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, and *Weissella cibaria* P26 strains, and may preferably include *Weissella cibaria* CHK903, *Weissella cibaria* SGW054, and *Weissella cibaria* SPW2014.

The pharmaceutical composition for preventing or treating a neurodegenerative disease, and the food composition and the animal feed composition for preventing or ameliorating a neurodegenerative disease, comprising the *Weissella cibaria* strain of the present invention are characterized by inhibiting the growth of *Proteus mirabilis,* and have a protective effect against neurotoxicity-induced apoptosis of neurons and an inhibitory effect on aggregation of α-synuclein.

The pharmaceutical composition for preventing or treating a neurodegenerative disease, and the food composition and the animal feed composition for preventing or ameliorating a neurodegenerative disease, comprising the *Weissella cibaria* strain of the present invention have safety by not exhibiting any one or more activities from the group consisting of antibiotic resistance, antibiotic resistance gene, toxicity gene, hemolytic activity, mucin degradation ability, gelatin liquefaction ability, urease activity, indole production ability, beta-glucuronidase (β-glucuronidase) activity, cytotoxicity, bile salt deconjugation activity, and biogenic amine production ability.

The degenerative disease of the present invention includes Parkinson's disease, Alzheimer's disease, Pick's disease, Huntington's disease, Lou Gehrig's disease, prion disease, Lewy body dementia, multiple system atrophy, progressive supranuclear palsy, Friedreich's ataxia, temporal lobe epilepsy, and stroke. Preferably, it may be Parkinson's disease.

The present invention provides a *Weissella cibaria* CHK903 strain (accession number: KCCM13224P).

The present invention provides a *Weissella cibaria* SGW054 strain (accession number: KCCM13225P).

The present invention provides a *Weissella cibaria* SPW2014 strain (accession number: KCCM13226P).

The present invention provides a method for treating a neurodegenerative disease, comprising administering a composition comprising a *Weissella cibaria* strain to a subject with a neurodegenerative disease.

The present invention provides a composition comprising a *Weissella cibaria* strain, for use in the prevention or treatment of a neurodegenerative disease.

The present invention provides the use of a composition comprising a *Weissella cibaria* strain, for the prevention or treatment of a neurodegenerative disease.

### Effects of Invention

The novel *Weissella cibaria* strain of the present invention and a composition comprising the same have not only antimicrobial activity against *Proteus mirabilis,* but also protective ability against neurotoxicity-induced apoptosis of neurons, inhibition ability against aggregation of α-synuclein, and safety, and thus can be utilized as a pharmaceutical composition, a food composition, and an animal feed composition, which are excellent in prevention, amelioration, or treatment of neurodegenerative diseases.

### Brief Description of Drawings

Fig. 1 shows cell viability by *Weissella cibaria* strains in brain neurons.
Fig. 2 shows the level of α-synuclein produced by *Weissella cibaria* strains in intestinal secretion cells.
Fig. 3 shows the level of α-synuclein for each concentration by killed microbial cells of *Weissella cibaria* strains in intestinal secretion cells.
Fig. 4 shows the results of the E-test experiment of a *Weissella cibaria* SPW2014 strain.
Fig. 5 shows the results of the E-test experiment of a *Weissella cibaria* SGW054 strain.
Fig. 6 shows the results of the E-test experiment of a *Weissella cibaria* CHK903 strain.
Fig. 7 shows the results of an experiment confirming the β-type hemolytic activity of *Weissella cibaria* strains.
Fig. 8 shows the results of an experiment confirming the mucin degradation of *Weissella cibaria* strains.
Fig. 9 shows the results of an experiment confirming the gelatin liquefaction of *Weissella cibaria* strains.
Fig. 10 shows the results of an experiment confirming the urease activity of *Weissella cibaria* strains.
Fig. 11 shows the results of an experiment confirming the indole production of *Weissella cibaria* strains.
Fig. 12 shows cell viability for confirming the cytotoxicity of *Weissella cibaria* strains.
Fig. 13 shows the results of an experiment confirming the bile salt hydrolase activity of *Weissella cibaria* strains.
Fig. 14 shows the HPLC chromatogram results for detection of biogenic amines in *Weissella cibaria* strains.

### Best Mode for Carrying out the Invention

Hereinafter, with reference to the accompanying drawings, embodiments and examples of the present invention will be described in detail so that those of ordinary skill in the art to which the present invention belongs can easily carry out. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention provides a novel *Weissella cibaria* strain and a composition for the prevention, amelioration, or treatment of a neurodegenerative disease comprising the same.

The *Weissella cibaria* is a relatively recently discovered lactic acid bacterium and is found in various foods, including fermented foods. It is mainly found in Korean kimchi, is one of the dominant species of kimchi, and is known to be involved in the early stages of kimchi fermentation. It has also been reported to produce relatively small amounts of lactic acid and produce functional extracellular polysaccharides.

The *Weissella cibaria* strain of the present invention may be *Weissella cibaria* CHK903, *Weissella cibaria* SGW054, *Weissella cibaria* SPW2014, *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, or *Weissella cibaria* P26, but is not limited thereto.

The *Weissella cibaria* CHK903 strain of the present invention was deposited on August 5, 2022 at the Korean Culture Center of Microorganisms (accession number: KCCM13224P).

The *Weissella cibaria* SGW054 strain of the present invention was deposited on August 5, 2022 at the Korean Culture Center of Microorganisms (accession number: KCCM13225P).

The *Weissella cibaria* SPW2014 strain of the present invention was deposited on August 5, 2022 at the Korean Culture Center of Microorganisms (accession number: KCCM13226P).

The *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, and *Weissella cibaria* P26 strains of the present invention have antimicrobial activity against *Proteus mirabilis* and excellent effect on a neurodegenerative disease, and the culture conditions, preservation conditions, restoration conditions, and survival test conditions are as follows.

### <Culture conditions>

- Medium composition: MRS (1.0 % peptone, 1.0 % beef extract, 0.4 % yeast extract, 2.0 % glucose, 0.5 % sodium acetate trihydrate, 0.1 % polysorbate 80, 0.2 % dipotassium hydrogen phosphate, 0.2 % triammonium citrate, 0.02 % magnesium sulfate heptahydrate, 0.005 % manganese sulfate tetrahydrate)
- culture temperature: 37 °C
- medium pH: pH 6.2
- culture time: 24 hours
- oxygen requirements: facultative anaerobic
- liquid stationary culture

### <Preservation conditions>

- freeze drying
- dispersant: composition 10% skim milk, pH 6.8, sterilization conditions 121 °C, 15 minutes
- vacuum degree: 5 to 10 mmHg
- freezing temperature: -40 to -50 °C

### <Restoration conditions>

- restoration agent: composition MRS medium, pH 6.2, sterilization conditions 121 °C, 15 minutes
- restoration temperature: 37 °C

### <Survival test conditions>

- suspend freeze-dried microbial cell powder using PBS (phosphate buffered saline), leave for 15 minutes, inoculate on a MRS liquid medium and a MRS agar plate, and then culture at 37 °C for 24 hours.

In one embodiment, the *Weissella cibaria* strain may be isolated from kimchi or tangerines.

The neurodegenerative disease may be Parkinson's disease, Alzheimer's disease, Pick's disease, Huntington's disease, Lou Gehrig's disease, prion disease, Lewy body dementia, multiple system atrophy, progressive supranuclear palsy, Friedreich's ataxia, temporal lobe epilepsy, and stroke, and preferably Parkinson's disease, but is not limited thereto.

The *Weissella cibaria* strain of the present invention and a composition for the prevention, amelioration, or treatment of a neurodegenerative disease comprising the same have antimicrobial activity against *Proteus mirabilis,* protective ability against neurotoxicity-induced apoptosis of neurons, inhibition ability against aggregation of α-synuclein, and safety.

The *Proteus mirabilis* is a Gram-negative rod bacterium belonging to the *Enterobacteriaceae* family and is known to be a strain that biosynthesizes lipopolysaccharide (LPS), which causes inflammation. In addition, as disclosed in Korean Patent Registration No. 10-1860566, it has been proven that *Proteus mirabilis* is directly involved in causing Parkinson's disease.

The neurotoxicity or neurotoxin is a toxin that acts specifically on neurons, synapses, or the nervous system as a whole, and is a substance that causes damage to brain structures and induces chronic diseases. Neurotoxins include, for example, adrenergic neurotoxins, cholinergic neurotoxins, dopaminergic neurotoxins, excitotoxins and other neurotoxins. Examples of adrenergic neurotoxins include N-(2-chloroethyl)-N-ethyl-2-bromobenzylamine hydrochloride, and examples of cholinergic neurotoxins include acetyl ethyl choline mustard hydrochloride. Examples of dopaminergic neurotoxins include 6-hydroxydopamine HBr (6-OHDA), 1-methyl-4-(2-methylphenyl)-1,2,3,6-tetrahydro-pyridine hydrochloride, 1-methyl-4-phenyl-2,3-dihydropyridinium perchlorate, N-methyl-4-phenyl-1,2,5,6-tetrahydropyridine HCl (MPTP), 1-methyl-4-phenylpyridinium iodide (MPP+), paraquat, and rotenone; and examples of excitotoxins include NMDA and kainic acid. The MPTP, MPP+, paraquat, rotenone, and 6-OHDA are known to induce Parkinson's disease-like symptoms in animal models.

The alpha-synuclein (α-synuclein) is a protein that aids neurotransmission between brain cells, and it is known that overexpression of this protein is the main cause of Parkinson's disease.

The present invention provides a *Weissella cibaria* strain and a pharmaceutical composition for preventing or treating a neurodegenerative disease comprising the same.

The *Weissella cibaria* strain and the neurodegenerative disease are as described above, and the strain may be a culture solution, a killed microbial cell, an internal factor, or a microbial cell.

In one embodiment, the pharmaceutical composition may be formulated into powders, granules, tablets, coated tablets, pills, sugar-coated tablets, capsules, solutions, suspensions, gels, syrups, slurries, suppositories, emulsions, pastes, ointments, creams, lotions, powders, sprays, or suspensions. Excipients such as fillers, extenders, binders, wetting agents, disintegrants, surfactants, and diluents commonly used for the formulation may additionally be included. In addition to simple excipients, lubricants such as magnesium stearate and talc may also be used, but are not limited thereto.

The present invention provides a *Weissella cibaria* strain and a food composition for preventing or ameliorating a neurodegenerative disease comprising the same.

The *Weissella cibaria* strain and the neurodegenerative disease are as described above, and the strain may be a culture solution, a killed microbial cell, an internal factor, or a microbial cell.

In one embodiment, the food composition may be a health functional food, a dairy product, a fermented product, or a food additive.

In one embodiment, the food composition includes the form of pills, powders, granules, precipitates, tablets, capsules, solutions, pastes, gels, or jellies, and for the food compositions of each dosage form, those of ordinary skill in the art can appropriately select and combine the ingredients commonly used in the field in addition to the active ingredients depending on the formulation or purpose of use without difficulty.

The present invention provides a *Weissella cibaria* strain and an animal feed composition for preventing or ameliorating a neurodegenerative disease comprising the same.

The *Weissella cibaria* strain and the neurodegenerative disease are as described above, and the strain may be a culture solution, a killed microbial cell, an internal factor, or a microbial cell.

The animal feed composition, the feed composition, or the feed is food given to animals, and refers to a substance that supplies organic or inorganic nutrients necessary for maintaining the life of animals and raising animals.

In one embodiment, the animal feed composition may include nutrients such as energy, protein, lipid, vitamins, and minerals required by the subject (animal) that consumes the feed.

As used herein, the term "prevention" refers to any act that suppresses or delays the onset of a disease by administration of a composition, and "amelioration" refers to any action that results in at least a reduction in the severity of a parameter associated with a condition, such as a symptom, by administration of a composition, and "treatment" refers to any action that improves or beneficially changes the symptoms of a subject suspected of having a disease and a subject who has developed a disease by administration of a composition.

Hereinafter, the present invention will be described in more detail through the examples, but the following examples are for illustrative purposes only and are not intended to limit the scope of the present invention.

### [Example 1]

### Screening of lactic acid bacteria exhibiting antimicrobial activity against Proteus mirabilis

The following experiment was performed to select lactic acid bacteria exhibiting antimicrobial activity against *Proteus mirabilis* among a total of 987 lactic acid bacteria.

### 1-1. Culture of lactic acid bacteria and preparation of culture supernatant

Lactic acid bacteria stored in a deep freezer (-80 °C) were thawed and grown on MRS agar medium, and then the resulting colonies were subcultured on new MRS agar medium (BD, Franklin Lakes, NJ, USA). The colonies of the subcultured lactic acid bacteria were inoculated into 1 mL of MRS broth, cultured at 37 °C for 20 hours, and then centrifuged at 14,000 Xg for 5 minutes to obtain the culture supernatant.

### 1-2. Culture of Proteus mirabilis

The *Proteus mirabilis* strain (METACEN THERAPEUTICS CO., LTD) was inoculated into nutrient broth (BD, Franklin Lakes, NJ, USA) and cultured with shaking at 200 rpm for 20 hours at 37 °C to prepare a culture solution.

### 1-3. First screening: Measurement of antimicrobial activity by paper disc method using top agar

Top agar was prepared by adding 0.8% agar to the nutrient medium and autoclaved. 100 µL of *Proteus mirabilis* culture solution was added and then overlaid on the nutrient agar medium. A sterilized paper disc (Advantec, Diameter: 6 mm) was placed thereon, 10 µL of lactic acid bacteria culture supernatant was added dropwise, and then cultured at 37 °C for 20 hours, and the resulting diameter of the growth inhibition ring of *Proteus mirabilis* was measured.

As a result of measuring the diameter of the growth inhibition ring of *Proteus mirabilis* for a total of 987 lactic acid bacteria, it was confirmed that most lactic acid bacteria strains did not show growth inhibitory activity against *Proteus mirabilis,* and only some lactic acid bacteria strains showed growth inhibitory activity. Therefore, 50 lactic acid bacteria showing the diameter of the growth inhibition ring of *Proteus mirabilis* of 10 mm or more were determined to be strains with excellent antimicrobial activity and were selected.

### 1-4. Second screening: Measurement of antimicrobial activity by paper disc method using McFarland solution

The antimicrobial activity of the lactic acid bacteria culture supernatant was measured repeatedly three times for the 50 lactic acid bacteria selected in the first screening by the paper disc method using McFarland solution. The paper disc method using McFarland solution is a method that can reduce errors in antimicrobial activity due to differences in the number of cells of the indicator strain because it can maintain a constant cell concentration of the indicator strain (*Proteus mirabilis*)*.*

As shown in Table 1 below, as a result of measuring the diameter of the growth inhibition ring of *Proteus mirabilis* for a total of 50 lactic acid bacteria, *Weissella cibaria* strains showed a significantly superior effect compared to other strains. In particular, The *Weissella cibaria* CHK903 strain showed the most excellent mean value of 16 mm, and the *Weissella cibaria* SGW054 and *Weissella cibaria* SPW2014 strains showed excellent mean value of 14 mm. In addition, the *Weissella cibaria* 200022, *Weissella cibaria* SFL001, *Weissella cibaria* JJW001, *Weissella cibaria* SLW6932, and *Weissella cibaria* P26 strains showed excellent mean value of 13 mm, and the *Weissella cibaria* FCK913, *Weissella cibaria* P8, and *Weissella cibaria* P9 strains showed excellent mean value of 12 mm.

**[Table 1]**

| Lactic acid bacteria | Strain No. | Diameter of growth inhibition ring (mm) | | | | |
|---|---|---|---|---|---|---|
| | | Top agar method | McFarland method | | | |
| | | | 1st | 2nd | 3rd | Mean |
| *Weissella cibaria* | 200022 | 10 | 10 | 16 | 13 | 13 |
| *Weissella cibaria* | SFL001 | 10 | 10 | 14 | 14 | 13 |
| *Weissella cibaria* | CHK903 | 15 | 15 | 14 | 18 | 16 |
| *Weissella cibaria* | FCK913 | 10 | 10 | 14 | 13 | 12 |
| *Weissella cibaria* | JJW001 | 10 | 10 | 14 | 15 | 13 |
| *Weissella cibaria* | SGW054 | 11 | 11 | 16 | 16 | 14 |
| *Weissella cibaria* | SLW6932 | 10 | 10 | 15 | 15 | 13 |
| *Weissella cibaria* | SPW2014 | 11 | 11 | 18 | 14 | 14 |
| *Weissella cibaria* | P8 | 10 | 10 | 13 | 12 | 12 |
| *Weissella cibaria* | P9 | 11 | 11 | 12 | 14 | 12 |
| *Weissella cibaria* | P26 | 12 | 12 | 15 | 12 | 13 |

In the results of the first (Top agar method) and second (McFarland method) screening, among strains that commonly show a mean value of 10 mm or more in the diameter of the growth inhibition ring, the strain having a high value in the results of the McFarland method, which can reduce the error in antimicrobial activity due to the difference in the number of cells of the indicator strain, was used as the standard for final selection, and the three strains that showed the highest mean value of 14 mm or more in the diameter of the growth inhibition ring were selected.

Therefore, as shown in Table 2 below, the *Weissella cibaria* CHK903, *Weissella cibaria* SGW054, and *Weissella cibaria* SPW2014 strains were selected as strains showing significantly excellent antimicrobial activity against *Proteus mirabilis,* and in the following examples, additional experiments were performed on the three *Weissella cibaria* strains.

In addition, the *Weissella cibaria CHK903 strain* was deposited on August 5, 2022 at the Korean Culture Center of Microorganisms (accession number: KCCM13224P), the *Weissella cibaria SGW054 strain* was deposited on August 5, 2022 at the Korean Culture Center of Microorganisms (accession number: KCCM13225P), and the *Weissella cibaria SPW2014 strain* was deposited on August 5, 2022 at the Korean Culture Center of Microorganisms (accession number: KCCM13226P).

**[Table 2]**

| Lactic acid bacteria | Strain No. | Diameter of growth inhibition ring (mm) | Source |
|---|---|---|---|
| *Weissella cibaria* | CHK903 | 16 | chive kimchi |
| *Weissella cibaria* | SGW054 | 14 | mustard kimchi |
| *Weissella cibaria* | SPW2014 | 14 | tangerine |

Additionally, as a result of an experiment with 72 lactic acid bacteria shown in Table 3 below, the following strains did not show a growth inhibitory effect on *Proteus mirabilis,* and it was found that *Weissella cibaria* showed a significantly excellent effect compared to other strains.

**[Table 3]**

| Lactic acid bacteria | Strain No. |
|---|---|
| Aerococcus urinaeequi | SLA2732 |
| Aerococcus urinaeequi | SAA371 |
| Enterococcus casseliflavus | SLE4331 |
| Enterococcus casseliflavus | SLE4333 |
| Enterococcus faecalis | JGE004 |
| Enterococcus faecalis | DDE005 |
| Enterococcus faecalis | SAE271 |
| Enterococcus faecium | SLE3232 |
| Enterococcus lactis | SGE022 |
| Enterococcus lactis | SLE3331 |
| Enterococcus raffinosus | SLE4332 |
| Enterococcus raffinosus | SAE411 |
| Enterococcus raffinosus | SAE415 |
| Staphylococcus xylosus | SLS5832 |
| Staphylococcus xylosus | STA111 |
| Staphylococcus xylosus | SLS3733 |
| Staphylococcus xylosus | SLS5831 |
| Staphylococcus xylosus | SLS5832 |
| Staphylococcus xylosus | SLS1632 |
| Staphylococcus xylosus | SLS1634 |
| Staphylococcus xylosus | SAS572 |
| Staphylococcus xylosus | SAS573 |
| Staphylococcus xylosus | SAS575 |
| Staphylococcus xylosus | SAS231 |
| Staphylococcus xylosus | SAS232 |
| Staphylococcus xylosus | SAS241 |
| Staphylococcus xylosus | SAS251 |
| Staphylococcus xylosus | SAS262 |
| Staphylococcus xylosus | SAS273 |
| Staphylococcus xylosus | SAS113 |
| Staphylococcus xylosus | SAS114 |
| Staphylococcus xylosus | SPS2512 |
| Staphylococcus xylosus | SPS5814 |
| Staphylococcus xylosus | SPS1111 |
| Staphylococcus xylosus | SPS1114 |
| Staphylococcus warneri | STA503 |
| Staphylococcus warneri | SLS1731 |
| Staphylococcus warneri | SLS1734 |
| Staphylococcus warneri | SLS1735 |
| Staphylococcus warneri | SLS1831 |
| Staphylococcus warneri | SLS2031 |
| Staphylococcus warneri | SLS2433 |
| Staphylococcus warneri | SLS3731 |
| Staphylococcus warneri | SLS3833 |
| Staphylococcus warneri | SLS3834 |
| Staphylococcus warneri | SAS621 |
| Staphylococcus warneri | SAS633 |
| Staphylococcus warneri | SAS261 |
| Staphylococcus warneri | SAS351 |
| Staphylococcus warneri | SLS2521 |
| Staphylococcus sciuri | STA302 |
| Staphylococcus sciuri | STA1203 |
| Staphylococcus pasteuri | SLS1732 |
| Staphylococcus pasteuri | SLS1736 |
| Staphylococcus pasteuri | SLS3732 |
| Staphylococcus pasteuri | SLS3831 |
| Staphylococcus hominis | SLS1633 |
| Staphylococcus epidermidis | DDS001 |
| Staphylococcus epidermidis | SLS1733 |
| Staphylococcus epidermidis | SLS2032 |
| Staphylococcus epidermidis | SLS2033 |
| Staphylococcus epidermidis | SAS272 |
| Carnobacterium divergens | SAC583 |
| Carnobacterium divergens | SPC5811 |
| Vagococcus carniphilus | SLV1832 |
| Vagococcus carniphilus | SAV182 |
| Vagococcus carniphilus | SAV183 |
| Vagococcus carniphilus | SAV184 |
| Vagococcus carniphilus | SAV185 |
| Vagococcus carniphilus | SAV263 |
| Vagococcus fluvialis | SAV181 |
| Wautersiella sp. | HBW103 |

### [Example 2]

### Confirmation of antimicrobial activity of Weissella cibaria strain against Proteus mirabilis

The following experiment was performed to confirm the antimicrobial activity against *Proteus mirabilis* for the *Weissella cibaria* strains distributed from the World Institute of Kimchi (KCKM) and the Korea Federation of Culture Collection (KCCM).

The colonies of the lactic acid bacteria grown on MRS agar medium (BD, Franklin Lakes, NJ, USA) were inoculated into 1 mL of MRS broth, stationary cultured at 37 °C for 20 hours, and then centrifuged at 14,000 Xg for 5 minutes to prepare the culture supernatant. The *Proteus mirabilis* strain was inoculated into nutrient broth (BD, Franklin Lakes, NJ, USA) and cultured with shaking at 200 rpm for 20 hours at 37 °C to prepare a culture solution. The colonies of *Proteus mirabilis* generated in a nutrient agar medium were suspended in saline solution (0.85 % NaCl) so that the turbidity was the same as that of the 0.5 McFarland standard solution, and the cell concentration was adjusted to 1.5 × 10⁸ CFU/mL. 50 µL of this solution was plated on the nutrient agar medium, and then a paper disc (Advantec, Diameter: 6 mm) was placed thereon, and 10 µL of lactic acid bacteria culture supernatant was added dropwise, and then cultured at 37 °C for 20 hours, and the resulting diameter of the growth inhibition ring of *Proteus mirabilis* was measured.

The results of the experiment are shown in Table 4 below.

**[Table 4]**

| Lactic acid bacteria | Strain No. | Diameter of growth inhibition ring (mm) |
|---|---|---|
| *Weissella cibaria* | KCKM 0331 | 12.5±0.7 |
| *Weissella cibaria* | KCKM 0332 | 11.0±0.0 |
| *Weissella cibaria* | KCKM 0334 | 15.0±1.4 |
| *Weissella cibaria* | KCKM 0339 | 11.0±1.4 |
| *Weissella cibaria* | KCKM 0342 | 13.5±0.7 |
| *Weissella cibaria* | KCKM 0344 | 14.5±0.7 |
| *Weissella cibaria* | KCKM 0350 | 12.0±1.4 |
| *Weissella cibaria* | KCKM 0358 | 11.0±1.4 |
| *Weissella cibaria* | KCKM 0360 | 10.5±0.7 |
| *Weissella cibaria* | KCKM 0361 | 12.00±0.0 |
| *Weissella cibaria* | KCCM 41287 | 14.0±1.4 |
| *Weissella cibaria* | KCCM 42079 | 15.7±0.5 |
| *Weissella cibaria* | KCCM 43206 | 15.7±0.5 |
| *Weissella cibaria* | KCCM 43207 | 12.7±0.5 |
| *Weissella cibaria* | CHK903 | 12.8±1.1 |

As shown in Table 4 above, not only the *Weissella cibaria* CHK903 strain of the present invention, but also the remaining 14 *Weissella cibaria* strains all had the diameter of the growth inhibition ring of 10 mm or more.

Therefore, it was confirmed that the *Weissella cibaria* strain shows excellent antimicrobial activity against *Proteus mirabilis.*

### [Example 3]

### Evaluation of protective ability against neurotoxicity-induced apoptosis of neurons in brain neurons

The following experiment was performed to evaluate the protective ability against apoptosis of neurons caused by MPP ⁺, a Parkinson's disease-inducing toxicity, in differentiated SH-Sy5y cells (brain neurons) for the *Weissella cibaria* strains shown in Table 2 above.

The SH-sy5y cell line was dispensed into each well of a 96 well plate in the number of 1 × 10⁴ cells and differentiated to exhibit dopaminergic neuron traits through retinoic acid treatment for 7 days. Thereafter, the existing medium was removed and replaced with 0.5% fetal bovine serum DMEM medium to which a sample was added at various concentrations. After 4 hours, the cells were treated with MPP ⁺ neurotoxicity at a concentration of 1 mM and cultured for an additional 44 hours. Thereafter, the medium was treated with the WST-1 solution and cultured for 2 hours to induce a reduction reaction, and then the absorbance was measured at 450 nm using an ELISA microplate reader.

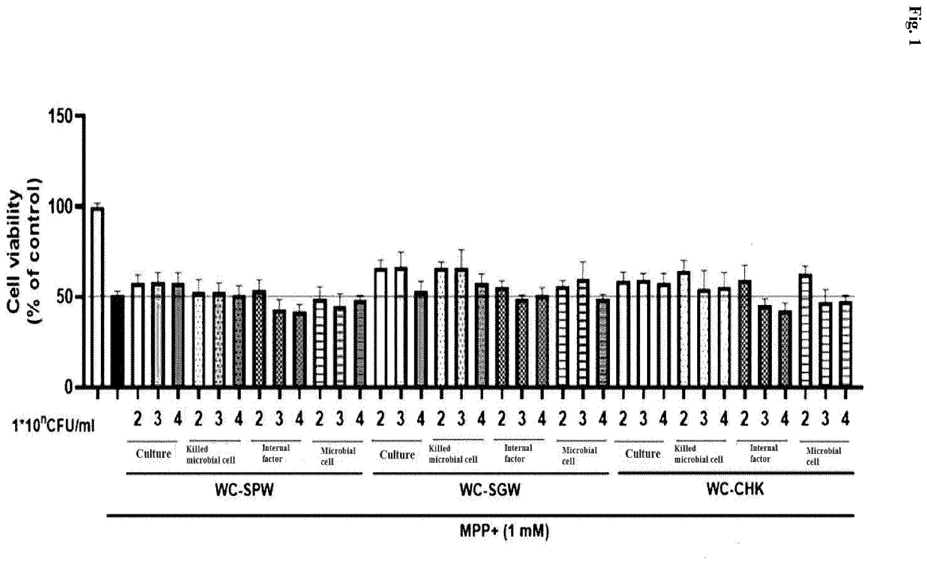

The cell viability based on the above experimental results are shown in Table 5 below and Fig. 1.

**[Table 5]**

| Cell viability | | | % compared to control | % compared to MPP⁺ | S.E.M. |
|---|---|---|---|---|---|
| Control | | | 100.00 | - | 1.64 |
| MPP⁺ | | | 52.98 | 100.00 | 4.23 |
| *Weissella cibaria* SPW2014 (WC-SPW) | Culture | 1 x 10² | 57.98 | 109.43 | 4.73 |
| | | 1 x 10³ | 58.23 | 109.91 | 6.54 |
| | | 1 x 10⁴ | 57.38 | 108.3 | 7.34 |
| | Killed microbial cell | 1 x 10² | 52.57 | 99.23 | 8.54 |
| | | 1 x 10³ | 52.39 | 98.89 | 6.44 |
| | | 1 x 10⁴ | 50.72 | 95.73 | 6.68 |
| | Internal factor | 1 x 10² | 53.65 | 101.26 | 7.16 |
| | | 1 x 10³ | 43.17 | 81.48 | 6.27 |
| | | 1 x 10⁴ | 42.03 | 79.33 | 4.55 |
| | Microbial cell | 1 x 10² | 48.55 | 91.64 | 8.58 |
| | | 1 x 10³ | 44.62 | 84.23 | 8.58 |
| | | 1 x 10⁴ | 48.19 | 90.97 | 3.1 |
| *Weissella cibaria* SGW054 (WC-SGW) | Culture | 1 x 10² | 65.86 | 124.31 | 5.7 |
| | | 1 x 10³ | 66.67 | 125.83 | 9.78 |
| | | 1 x 10⁴ | 53.26 | 100.52 | 6.5 |
| | Killed microbial cell | 1 x 10² | 65.79 | 124.17 | 4.23 |
| | | 1 x 10³ | 66.32 | 125.18 | 11.76 |
| | | 1 x 10⁴ | 57.59 | 108.71 | 6.43 |
| | Internal factor | 1 x 10² | 55.25 | 104.29 | 4.37 |
| | | 1 x 10³ | 48.46 | 91.47 | 3.05 |
| | | 1 x 10⁴ | 50.9 | 96.07 | 5.09 |
| | Microbial cell | 1 x 10² | 55.9 | 105.51 | 3.68 |
| | | 1 x 10³ | 60.11 | 113.45 | 11.44 |
| | | 1 x 10⁴ | 48.41 | 91.37 | 3.69 |
| *Weissella cibaria* CHK903 (WC-CHK) | Culture | 1 x 10² | 58.7 | 110.79 | 6.27 |
| | | 1 x 10³ | 59.14 | 111.62 | 4.83 |
| | | 1 x 10⁴ | 57.4 | 108.35 | 6.94 |
| | Killed microbial cell | 1 x 10² | 64.18 | 121.14 | 6.75 |
| | | 1 x 10³ | 54.49 | 102.85 | 12.38 |
| | | 1 x 10⁴ | 55.26 | 104.3 | 10.13 |
| | Internal factor | 1 x 10² | 59.2 | 111.74 | 10.35 |
| | | 1 x 10³ | 45.31 | 85.53 | 4.11 |
| | | 1 x 10⁴ | 42.53 | 80.28 | 4.97 |
| | Microbial cell | 1 x 10² | 62.92 | 118.77 | 5.29 |
| | | 1 x 10³ | 47.07 | 88.84 | 8.36 |
| | | 1 x 10⁴ | 47.43 | 89.52 | 4.16 |

As shown in Table 5 above and Fig. 1, the *Weissella cibaria* strains showed a higher cell viability compared to the control group at various concentrations of culture, killed microbial cells, and microbial cells. In addition, the *Weissella cibaria* strains showed a cell viability that was more than 5% higher than the level of MPP ⁺ induced apoptosis at various concentrations.

Therefore, it was confirmed that the *Weissella cibaria* strain of the present invention has an excellent protective effect against apoptosis of neurons caused by neurotoxicity (MPP ⁺).

### [Example 4]

### Evaluation 1 of inhibition ability against aggregation of α-synuclein induced by Proteus mirabilis in intestinal secretion cells

The following experiment was performed to evaluate the inhibition ability against aggregation of α-synuclein induced by *Proteus mirabilis* in STC-1 cells (intestinal secretion cells) for the *Weissella cibaria* strains shown in Table 2 above.

The STC-1 cell line was dispensed into a 60 mm culture plate in the number of 1 × 10⁶ cells. After 48 hours, the existing medium was removed and replaced with DMEM medium to which evaluation samples and killed PM cells were added, and culturing was performed for another 48 hours, and then the cells were obtained. The obtained cells were lysed in 1 × PBS containing protease inhibitors, and then the supernatant and pellet were separated by centrifugation at 12000 rpm for 10 minutes. The remaining pellet was electrophoresed twice on a 10% SDS-polyacrylamide gel, and then an alpha-synuclein primary antibody (α-synuclein) was added and left at 4 °C. After 12 hours, a secondary antibody (HRP conjugate) was added and reacted at room temperature for 1 hour, and then the film was developed by exposure to ECL. As a housekeeping protein, β-actin with a molecular weight of 43 kDa was used for comparison.

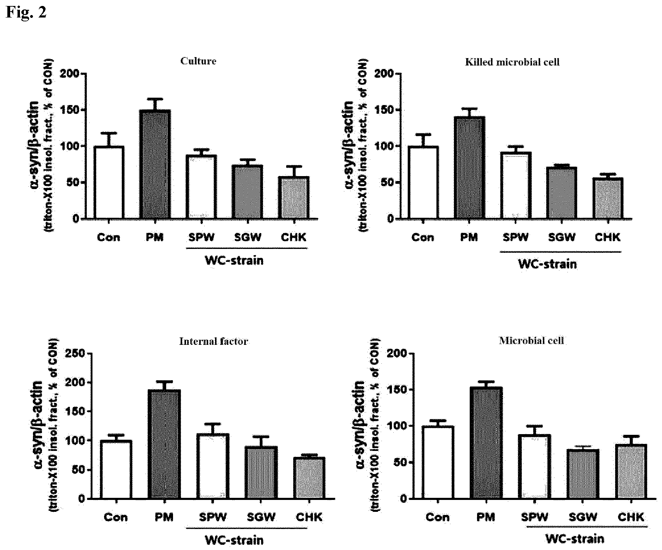

The results of the experiment are shown in Table 6 below and Fig. 2.

**[Table 6]**

| α-synuclein aggregate | | Control | *Proteus mirabilis* | *Weissella cibaria* SPW2014 (WC-SPW) | *Weissella cibaria* SGW054 (WC-SGW) | *Weissella cibaria* CHK903 (WC-CHK) |
|---|---|---|---|---|---|---|
| Culture | % compared to control | 100.00 | 150.20 | 88.60 | 74.35 | 58.36 |
| | % compared to PM | - | 100.00 | 58.99 | 49.51 | 38.86 |
| | S.E.M | 18.48 | 14.47 | 7.41 | 7.67 | 14.43 |
| Killed microbial cell | % compared to control | 100.00 | 140.96 | 92.73 | 71.32 | 56.85 |
| | % compared to PM | - | 100.00 | 65.79 | 50.60 | 40.34 |
| | S.E.M | 16.31 | 10.58 | 7.78 | 3.26 | 5.66 |
| Internal factor | % compared to control | 100.00 | 187.44 | 112.20 | 91.23 | 72.43 |
| | % compared to PM | - | 100.00 | 59.85 | 48.67 | 38.64 |
| | S.E.M | 9.17 | 14.03 | 16.34 | 15.54 | 3.94 |
| Microbial cell | % compared to control | 100.00 | 154.30 | 88.53 | 68.77 | 75.13 |
| | % compared to PM | - | 100.00 | 57.37 | 44.56 | 48.69 |
| | S.E.M | 7.35 | 6.85 | 11.96 | 4.57 | 10.79 |

As shown in Table 6 above and Fig. 2, the *Weissella cibaria* strains showed less α-synuclein compared to the control group in all of the culture, killed microbial cells, internal factor, and microbial cells. In addition, it was shown that the aggregation of α-synuclein was improved to a level of up to about 61% compared to *Proteus mirabilis.*

Therefore, it was confirmed that the *Weissella cibaria* strain of the present invention has an excellent inhibitory effect on the aggregation of α-synuclein induced by *Proteus mirabilis.*

### [Example 5]

### Evaluation 2 of inhibition ability against aggregation of α-synuclein induced by Proteus mirabilis in intestinal secretion cells

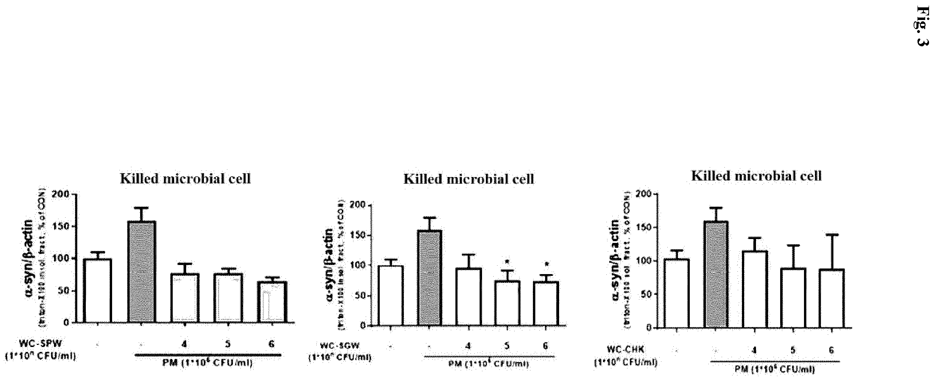

An experiment was performed in the same manner as in Example 4 to evaluate the inhibition ability of killed microbial cells for each concentration against the aggregation of α-synuclein induced by *Proteus mirabilis* in STC-1 cells (intestinal secretion cells) for the *Weissella cibaria* strains shown in Table 2 above, and the results are shown in Table 7 below and Fig. 3.

**[Table 7]**

| α-synuclein aggregate | | Control | *Proteus mirabilis* | 1 x 10⁴ | 1 x 10⁵ | 1 x 10⁶ |
|---|---|---|---|---|---|---|
| *Weissella cibaria* SPW2014 (WC-SPW) | % compared to control | 100 | 159.74 | 77.57 | 77.86 | 64.96 |
| | % compared to PM | - | 100.00 | 48.56 | 48.74 | 40.67 |
| | S.E.M | 9.47 | 19.55 | 11.86 | 5.45 | 5.34 |
| | % compared to control | 100 | 159.74 | 96.84 | 75.51 | 74.89 |
| *Weissella cibaria* SGW054 (WC-SGW) | % compared to PM | - | 100.00 | 60.62 | 47.27 | 46.88 |
| | S.E.M | 9.47 | 19.55 | 17.74 | 13.38 | 8.36 |
| *Weissella cibaria* CHK903 (WC-CHK) | % compared to control | 100 | 159.74 | 116.22 | 89.99 | 88.19 |
| | % compared to PM | - | 100.00 | 72.75 | 56.34 | 55.21 |
| | S.E.M | 9.47 | 19.55 | 15.04 | 27.12 | 41.98 |

As shown in Table 7 above and Fig. 3, the killed microbial cells of the *Weissella cibaria* strains were shown to reduce the aggregation of α-synuclein in a dose-dependent manner, and the aggregation of α-synuclein was shown to be improved to a level of up to about 59 % compared to *Proteus mirabilis.*

Therefore, it was confirmed that the *Weissella cibaria* strain of the present invention has an excellent inhibitory effect on the aggregation of α-synuclein induced by *Proteus mirabilis.*

### [Example 6]

### Safety evaluation 1: antibiotic resistance and toxicity

If lactic acid bacteria have an antibiotic resistance gene, it is dangerous because the antibiotic may not be effective during bacterial infection, and the transfer of the antibiotic resistance gene may occur in the intestines. Therefore, it is of utmost importance to understand the characteristics of antibiotic resistance in microorganisms.

### 6-1. Antibiotic resistance (E-test)

The following experiment was performed to evaluate the antibiotic resistance For the *Weissella cibaria* strains shown in Table 2 above.

The colonies of the strain grown on the MRS agar plate were taken and inoculated into MRS broth, and then cultured at 37 °C for 20 hours, and then 100 µL of the culture solution was adjusted to the same turbidity as that of the 3 McFarland standard (0.03% barium chloride, 5.97% sulfuric acid), and plated on the MRS agar plate without antibiotics added. One type of E-test strip was placed in the center on the plate and cultured at 37 °C for 72 hours, and then the number at the end of the strip indicated by the inhibition ring was determined as the minimum inhibitory concentration (MIC). The investigated antibiotics were a total of 9 types: ampicillin, vancomycin, gentamycin, kanamycin, streptomycin, erythromycin, clindamycin, tetracycline, and chloramphenicol. The MIC cut-off value of each antibiotic was evaluated by applying the value proposed by Jeong & Lee (2015).

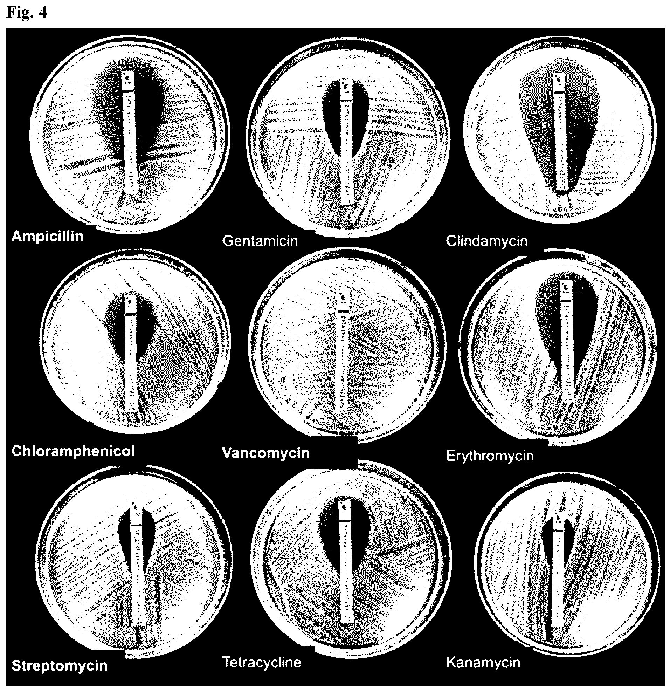

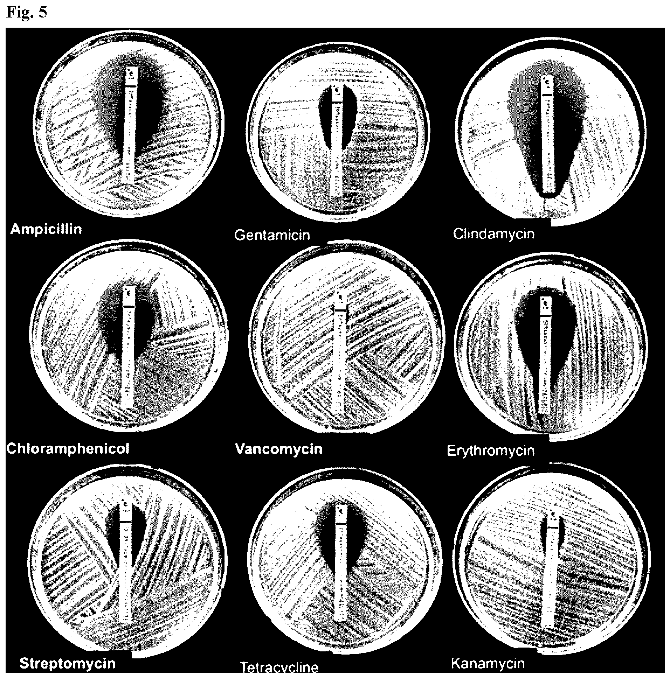

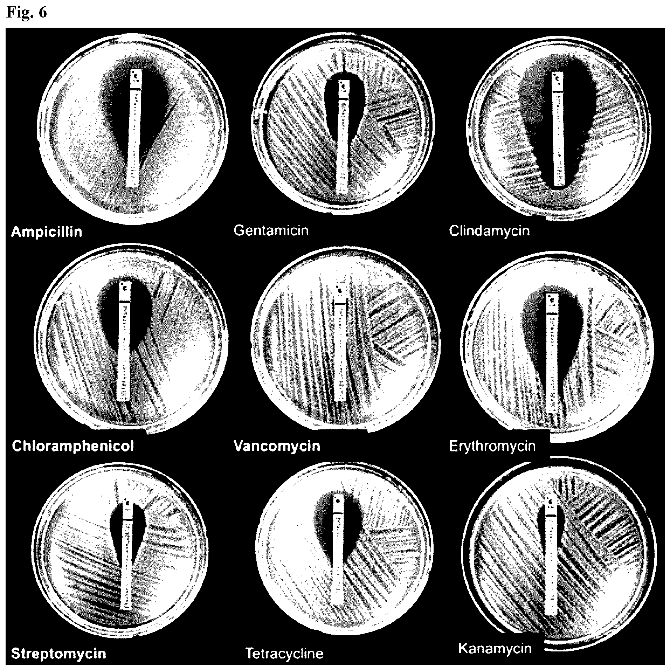

The results of the experiment are shown in Figs. 4 to 6, and the measured MIC values (µg/mL) are shown in Table 8 below.

**[Table 8]**

| Antibiotic | Cut-off value | *Weissella cibaria* SPW2014 | *Weissella cibaria* SGW054 | *Weissella cibaria* CHK903 |
|---|---|---|---|---|
| ampicillin | 2.00 | 0.25 | 0.19 | 0.25 |
| vancomycin | n.r.* | - | - | - |
| gentamycin | 32.00 | 1.5 | 2.00 | 3.00 |
| kanamycin | n.a.** | 8.00 | 6.00 | 16.00 |
| streptomycin | 64.00 | 12.00 | 8.00 | 8.00 |
| erythromycin | 1.00 | 0.19 | 0.19 | 0.125 |
| clindamycin | n.a. | 0.016 | 0.016 | 0.016 |
| tetracycline | 16.00 | 1.50 | 2.00 | 1.50 |
| chloramphenicol | 8.00 | 3.00 | 1.50 | 2.00 |
| *n.r., not required, **n.a., not available | | | | |

As shown in Table 8 above and Figs. 4 to 6, the MIC value of the *Weissella cibaria* strains was lower than the Cut-off value. In the case of vancomycin, resistance was not evaluated because many lactic acid bacteria species, including the Weissella genus, have intrinsic resistance. In the case of kanamycin, the *Weissella cibaria* strains showed a range of 6 to 16 µg/mL, and it was confirmed that they showed a lower value since the cut-off value of most lactic acid bacteria was 16 µg/mL or more. In addition, in the case of clindamycin, it was confirmed that all of the *Weissella cibaria* strains showed a very low MIC value of 0.016 µg/mL.

Therefore, it was confirmed that the *Weissella cibaria* strain of the present invention does not exhibit antibiotic resistance.

### 6-2. Antibiotic resistance gene

The following experiment was performed to confirm the presence or absence of an antibiotic resistance gene for the *Weissella cibaria* strains shown in Table 2 above.

As resistance genes, ampicillin (blaZ, bla), chloramphenicol (catA, cat), clindamycin (Inu(A), Inu(B)), erythromycin (ereA, ereB, Erm(B), Erm(B)-1, Erm(C)), gentamycin (aac(6')-aph(2"), aac(6')-aph(2"La)), kanamycin (aph(3')-I, aph(3')-III), streptomycin (aadA, aadE, ant(6)), tetracycline(tet(M), tet(M)-1, tet(K), tet(K)-1, tet(K)-2, tet(Q)), vancomycin (vanE, vanX) genes were selected and analyzed.

Genomic DNA of lactic acid bacteria was isolated using AccuPrep^{®} Genomic DNA Extraction Kit from Bioneer. PCR was performed by My Cycler^{™} (Bio-Rad, Hercules, CA, USA) using HotStart^{®} Premix (Bioneer, Daejeon, Korea). 0.1 µg of genomic DNA of the strain was used as template DNA, and forward primer and reverse primer were added to 300 nM, respectively. The obtained PCR product was analyzed by agarose gel electrophoresis. As the electrophoresis buffer solution, 0.5 times the TAE buffer solution (40 mM Tris-acetate, pH 8.0, 1 mM EDTA) was used. Using a 1.5% (w/v) agarose horizontal gel to which 0.5 µg/mL of ethidium bromide was added, the DNA solution to which 10×gel loading buffer solution (0.25% bromophenol blue, 40% (w/v) sucrose) was added in an amount 1/10 times the amount of the sample was electrophoresed at 100 V for 30 to 40 minutes. Thereafter, it was observed under ultraviolet light with a wavelength of 254 nm. For photography, the Bio-Rad Gel Doc XR+ gel documentation system equipped with a UV-filter and an orange filter was used.

The results of the experiment are shown in Table 9 below.

**[Table 9]**

| Antibiotic | Gene | Primer sequence (5'→3') | Whether to detect |
|---|---|---|---|
| ampicillin | blaZ | ACTTCAACACCTGCTGCTTTC TAGGTTCAGATTGGCCCTTAG | X |
| | bla | CATARTTCCGATAATASMGCC CGTSTTTAACTAAGTATSGY | X |
| chloramphenicol | catA | GGATATGAAATTTATCCCTC CAATCATCTACCCTATGAAT | X |
| | cat | TTAGGTTATTGGGATAAGTTA GCATGRTAACCATCACAWAC | X |
| clindamycin | Inu(A) | GGTGGCTGGGGGTAGATGTATTAACTGG GCTTCTTTTGAAATACATGGTATTTTTCGATC | X |
| | Inu(B) | CCTACCTATTGTTTGTGGAA ATAACGTTACTCTCCTATTTC | X |
| erythromycin | ereA | AACACCCTGAACCCAAGGGACG CTTCACATCCGGATTCGCTCGA | X |
| | ereB | AGAAATGGAGGTTCATACTTACCA CATATAATCATCACCAATGGCA | X |
| | Erm(B) | GAAAAGGTACTCAACCAAATAAGTAACGGTACTTAAATTGTTTAC | X |
| | Erm(B)-1 | CATTTAACGACGAAACTGGCGGAACATCTGTGGTATGGCG | X |
| | Erm(C) | TCAAAACATAATATAGATAAAGCTAATATTGTTTAAATCGTCAAT | X |
| gentamycin | aac(6')-aph(2") | CCAAGAGCAATAAGGGCATA CACTATCATAACCACTACCG | X |
| | aac(6')-aph(2"La | CAGAGCCTTGGGAAGATGAAG CCTCGTGTAATTCATGTTCTGGC | X |
| kanamycin | aph(3')-I | AACGTCTTGCTCGAGGCCGCG GGCAAGATCCTGGTATCGGTCTGCG | X |
| | aph(3')-III | GCCGATGTGGATTGCGAAAA GCTTGATCCCCAGTAAGTCA | X |
| streptomycin | aadA | ATCCTTCGGCGCGATTTTG GCAGCGCAATGACATTCTTG | X |
| | aadE | ATGGAATTATTCCCACCTGA TCAAAACCCCTATTAAAGCC | X |
| | ant(6) | ACTGGCTTAATCAATTTGGGGCCTTTCCGCCACCTCACCG | X |
| tetracycline | tet(M) | GGTGAACATCATAGACACGC CTTGTTCGAGTTCCAATGC | X |
| | tet(M)-1 | GTTAAATAGTGTTCTTGGAG CTAAGATATGGCTCTAACAA | X |
| | tet(K) | TCGATAGGAACAGCAGTACAGCAGATCCTACTCCTT | X |
| | tet(K)-1 | TTAGGTGAAGGGTTAGGTCCGCAAACTCATTCCAGAAGCA | X |
| | tet(K)-2 | TTATGGTGGTTGTAGCTAGAAAAAAGGGTTAGAAACTCTTGAAA | X |
| | tet(Q) | AGCGTCAAAGGGGAATCACTATCCCGGCGGGGTTGGCAAATA | X |
| vancomycin | vanE | TGTGGTATCGGAGCTGCAG GTCGATTCTCGCTAATCC | X |
| | vanX | TCGCGGTAGTCCCACCATTCGTT AAATCATCGTTGACCTGCGTTAT | X |

As shown in Table 9 above, a total of 26 antibiotic resistance genes were not detected in any of the *Weissella cibaria* strains.

Therefore, it was confirmed that the *Weissella cibaria* strain of the present invention does not have an antibiotic resistance gene.

### 6-3. Toxicity gene

The following experiment was performed to confirm the presence or absence of a toxicity gene for the *Weissella cibaria* strains shown in Table 2 above.

As toxicity genes, gelatinase (gelE), cytolysin (cylA, cylB, cylM), aggregation substance (agg, asa1) genes were selected and analyzed, and an experiment was performed in the same manner as in Example 6-2 above.

The results of the experiment are shown in Table 10 below.

**[Table 10]**

| Toxicity factor | Gene | Primer sequence (5'→3') | Whether to detect |
|---|---|---|---|
| gelatinase | gelE | ACCCCGTATCATTGGTTT ACGCATTGCTTTTCCATC | X |
| cytolysin | cylA | TGGATGATAGTGATAGGAAGT TCTACAGTAAATCTTTCGTCA | X |
| | cylB | ATTCCTACCTATGTTCTGTTA AATAAACTCTTCTTTTCCAAC | X |
| | cylM | CTGATGGAAAGAAGATAGTATTGAGTTGGTCTGATTACATTT | X |
| aggregation substance | Agg | AAGAAAAAGAAGTAGACCAAC AAACGGCAAGACAAGTAAATA | X |
| | Asa1 | GCACGCTATTACGAACTATGA TAAGAAAGAACATCACCACGA | X |

As shown in Table 10 above, no toxicity genes were detected in any of the *Weissella cibaria* strains.

Therefore, it was confirmed that the *Weissella cibaria* strain of the present invention does not have a toxicity gene.

Ultimately, it can be seen that the *Weissella cibaria* strain of the present invention does not exhibit antibiotic resistance and does not have antibiotic resistance genes or toxicity genes, making it safe.

### [Example 7]

### Safety evaluation 2: hemolytic activity

If the strain has hemolytic activity, it destroys the host's red blood cells. Therefore, this must be confirmed and safety must be evaluated.

For the *Weissella cibaria* strains shown in Table 2 above, the following experiment was performed to confirm whether they had the hemolytic activity.

Lactic acid bacteria and Streptococcus pyogenes KCCM 11873, which was used as a positive control group, were streaked on MRS medium containing 5% sheep blood and then cultured at 37 °C for 48 hours. If a clear halo is formed around the colony after culturing, it is classified as a microorganism with β-type hemolytic activity.

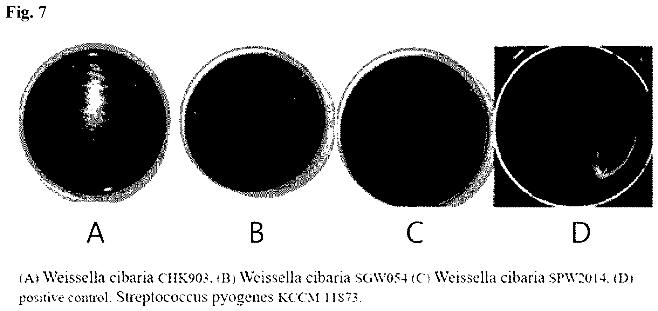

The results of the experiment are shown in Fig. 7. As shown in Fig. 7, it was found that no clear zone was formed in any of the *Weissella cibaria* strains.

Therefore, it was confirmed that the *Weissella cibaria* strain of the present invention does not have hemolytic activity and is safe.

### [Example 8]

### Safety evaluation 3: mucin degradation ability

For the *Weissella cibaria* strains shown in Table 2 above, the following experiment was performed to confirm whether they had the mucin degradation ability.

The seed culture solution of lactic acid bacteria was inoculated into 30 mL of basal MRS medium (MRS medium without glucose), basal MRS medium containing 0.3 % mucin (type III, Sigma), basal MRS medium containing 1 % glucose, and basal MRS medium containing 1 % glucose and 0.3 % mucin at a concentration of 1% and cultured at 37 °C for 24 hours. The culture solution was sampled at 8 and 24 hours after incubation. The growth rate of microorganisms was measured by measuring the absorbance at 600 nm with a spectrophotometer, and the pH was measured with a pH meter to confirm the change in pH according to the growth of microorganisms. If it has the mucin degradation ability, the pH is reduced as the strain grows using mucin as a carbon source.

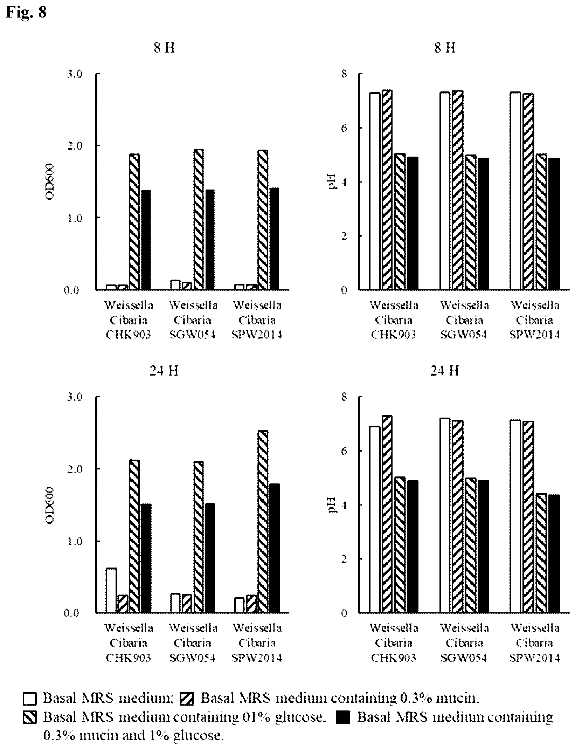

The results of the experiment are shown in Fig. 8. As shown in Fig. 8, it was found that in all of the *Weissella cibaria* strains, the absorbance was increased and the pH was decreased in the medium containing glucose and the medium containing glucose and mucin over time, while the absorbance was not increased and the pH was not decreased in the basal MRS medium and the medium containing mucin.

Therefore, it was confirmed that the *Weissella cibaria* strain of the present invention does not have the mucin degradation ability and is safe.

### [Example 9]

### Safety evaluation 4: gelatin liquefaction ability

For the *Weissella cibaria* strains shown in Table 2 above, the following experiment was performed to confirm whether they had the gelatin liquefaction ability.

Lactic acid bacteria were streaked in a tube containing MRS medium containing 12% gelatin, cultured at 37°C for 72 hours, and then left on ice for 30 minutes to observe whether they had the gelatin liquefaction ability. Brevibacillus parabrevis KCCM 41421 was used as a positive control group.

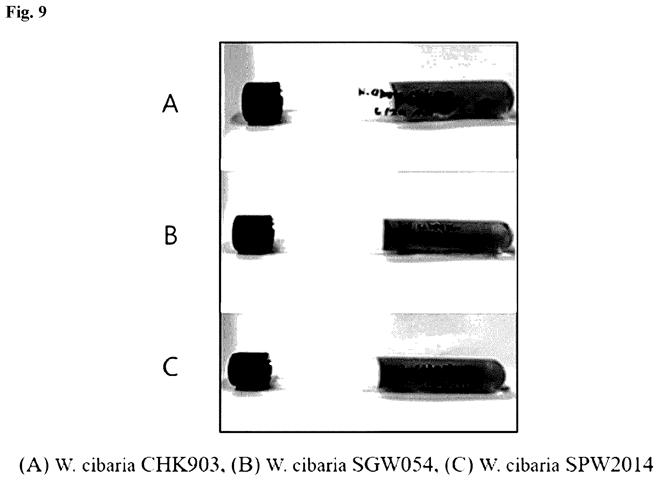

The results of the experiment are shown in Fig. 9. As shown in Fig. 9, none of the *Weissella cibaria* strains showed the gelatin liquefaction ability.

Therefore, it was confirmed that the *Weissella cibaria* strain of the present invention does not have the gelatin liquefaction ability and is safe.

### [Example 10]

### Safety evaluation 5: urease activity

For the *Weissella cibaria* strains shown in Table 2 above, the following experiment was performed to confirm whether they had the urease activity.

The urease activity was determined by changes in pH when the bacteria were inoculated into Christensen's urea agar (0.1 g peptone, 0.1 g dextrose, 0.5 g sodium chloride, 0.2 g KH2PO4, 2 g urea, 0.0012 g phenol red, 1.5 g agar, per 100 mL). Lactic acid bacteria and Proteus vulgaris KCCM 40211, which was used as a positive control group, were plated on a slope medium, cultured at 37 °C, and then changes in the color of the medium were confirmed after 6 hours, 24 hours, and 6 days of incubation.

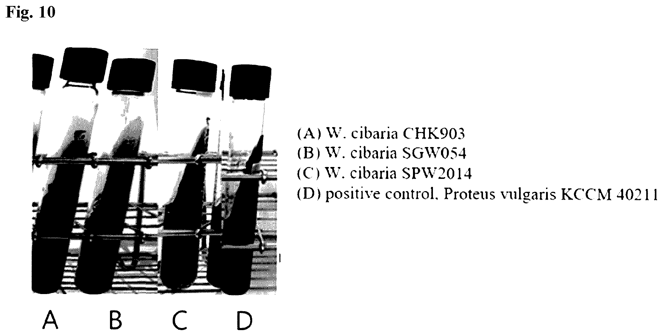

The results of the experiment are shown in Fig. 10. As shown in Fig. 10, none of the *Weissella cibaria* strains showed changes in the color of the medium until 6 days of incubation.

Therefore, it was confirmed that the *Weissella cibaria* strain of the present invention does not have the urease activityand is safe.

### [Example 11]

### Safety evaluation 6: indole production ability

For the *Weissella cibaria* strains shown in Table 2 above, the following experiment was performed to confirm whether they had the indole production ability.

Lactic acid bacteria and Escherichia coli ATCC 10536, which was used as a positive control group, were inoculated into Tryptophan medium (10.0 g casein enzyme hydrolysate, 5.0 g NaCl, 1.0 g DLtryptophan, per 1 L) and cultured at 37 °C for 18 hours. Thereafter, when 5 drops of Kovac's reagent (10 g p-dimethylaminobenzaldehyde, 150 mL butanol, and 50 mL hydrochloric acid) were added to the culture solution and it turned red, it was determined to be a positive bacterium.

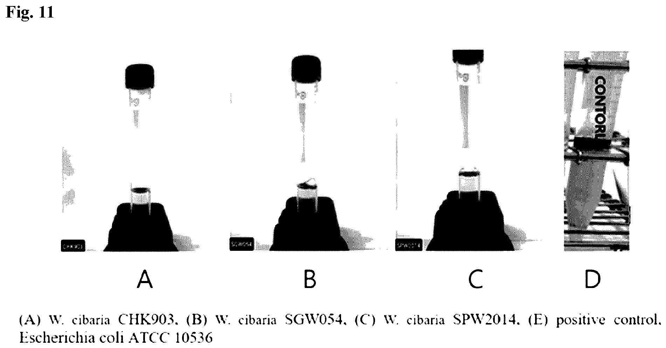

The results of the experiment are shown in Fig. 11. As shown in Fig. 11, none of the *Weissella cibaria* strains showed changes in the color of the culture solution.

Therefore, it was confirmed that the *Weissella cibaria* strain of the present invention does not have the indole production ability and is safe.

### [Example 12]

### Safety evaluation 7: β-glucuronidase activity

For the *Weissella cibaria* strains shown in Table 2 above, the following experiment was performed to confirm whether they had the β-glucuronidase activity.

The β-glucuronidase activity was measured using the API ZYM kit (bioMerieux, Marcy l'Etoile, France). Lactic acid bacteria were inoculated into MRS medium, cultured at 37 °C 24 hours, and then diluted in 2 mL sterile saline to adjust the turbidity of the culture solution to a turbidity corresponding to McFarland 5-6. 65 µL of this solution was dispensed onto an API strip and cultured at 37°C for 4 hours. Thereafter, ZYM A and ZYM B were added dropwise, and the presence or absence of enzyme activity was determined by color change after 5 minutes.

The results of the experiment are shown in Table 11 below.

**[Table 11]**

| Enzyme | *Weissella cibaria* CHK903 | *Weissella cibaria* SGW054 | *Weissella cibaria* SPW2014 |
|---|---|---|---|
| Alkaline phosphatase | - | - | - |
| Esterase (C4) | - | - | - |
| Esterase Lipase (C8) | - | - | - |
| Lipase (C14) | - | - | - |
| Leucine arylamidase | - | - | - |
| Valine arylamidase | - | - | - |
| Crystine arylamidase | - | - | - |
| Trypsin | - | - | - |
| α-Chymotrypsin | - | - | - |
| Acid phosphatase | + | + | + |
| Naphtol-AS-Bl-phosphohydrolase | + | + | + |
| α-Galactosidase | + | + | + |
| β-Galactosidase | - | - | - |
| β-Glucuronidase | - | - | - |
| α-Glucosidase | - | - | - |
| β-Glucosidase | - | - | - |
| N-Acetyl-β-glucosaminidase | - | - | - |
| α-Mannosidase | - | - | - |
| α-Fucosidase | - | - | - |

As shown in Table 11 above, none of the *Weissella cibaria* strains showed β-glucuronidase activity.

Therefore, it was confirmed that the *Weissella cibaria* strain of the present invention does not have the β-glucuronidase activity and is safe.

### [Example 13]

### Safety evaluation 8: cytotoxicity

If a strain produces toxins, it can cause harmful results to the human body. Therefore, the cytotoxicity of the strain must be confirmed through in vitro experiments based on animal cells. EZCYTOX is a reagent that measures the amount of living cells using water soluble tetrazolium (WST) and is used to measure cytotoxicity. WST reacts with cellular dehydrogenase to produce orange-colored water-soluble formazan. The dehydrogenase that reacts with WST is an enzyme present in the mitochondrial electron transport chain of metabolically active cells and is effective only in living cells. Therefore, the production of formazan has a linear correlation with the number of living cells, which can be seen by measuring the absorbance (450 nm).

For the *Weissella cibaria* strains shown in Table 2 above, the following experiment was performed to confirm whether they had the cytotoxicity.

Caco-2 cells were seeded in a 96-well plate at 5 × 10⁴ cells/well in DMEM medium supplemented with 10% fetal bovine serum (FBS) and 1% penicillin/streptomycin and then cultured in a 5% CO₂ incubator at 37 °C for 20 hours. Lactic acid bacteria were stationary cultured for 18 hours in MRS broth. As a positive control group, the culture solution of Klebsiella pneumoniae subsp. pneumoniae KCCM 41433 cultured in nutrient broth with shaking for 18 hours was used as a seed culture solution. 1% of each seed culture solution of lactic acid bacteria strains was inoculated into 5 mL of MRS medium and cultured at 37 °C for 3 hours, and then the culture solutionwas centrifuged at 20,781 Xg for 1 minute to harvest the cells. The harvested lactic acid bacteria cells were washed three times with DPBS and then suspended in 1 mL of DMEM medium supplemented with 10% FBS and 1% penicillin/streptomycin. The suspended lactic acid bacteria strain was added to Caco-2 cells so that the multiplicity of infection (MOI, number of viable lactic acid bacteria / number of Caco-2 cells) was 250, and then cultured at 37 °C, 5% CO₂ for 24 hours. After culturing, the cells were washed three times with DPBS, and then DMEM medium supplemented with 10% FBS and 1% penicillin/streptomycin was added at 200 µL/well, and then EZ-CYTOX was added at 20 µL/well, and reacted at 37 °C, 5% CO₂ for 30 minutes. Thereafter, the absorbance at 450 nm was measured using a microplate reader.

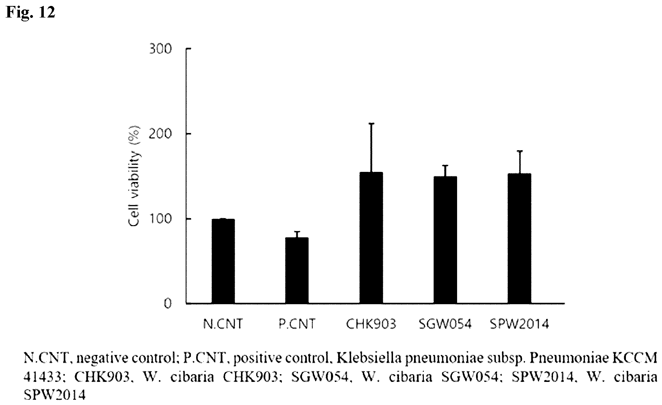

The results of the experiment are shown in Fig. 12. As shown in Fig. 12, the cell viability of the positive control group at MOI 250 was 80% or less, while all of the *Weissella cibaria* strains showed a cell viability of 80% or more.

Therefore, it was confirmed that the *Weissella cibaria* strain of the present invention does not have the cytotoxicity and is safe.

### [Example 14]

### Safety evaluation 9: Lactic acid production

Lactic acid has two optical isomers, L-form and D-form. In the human body, L-lactic acid can be metabolized, but the isomer D-lactic acid cannot be metabolized. Probiotic strains have DL-lactate racemase, an enzyme that converts L-lactate to D-lactate. If enzyme activity is strong, D-lactate may accumulate and cause acidosis in newborns, children, and patients with short bowel syndrome. Accordingly, WHO has recommended the daily intake of D-lactic acid to be less than 100 mg/kg body mass.

For the *Weissella cibaria* strains shown in Table 2 above, the following experiment was performed to confirm whether they produced D-lactic acid.

Lactic acid bacteria were cultured in MRS broth at 37 °C for 24 hours, and then the concentrations of L-lactic acid and D-lactic acid in the culture solution were measured using a D-lactate assay kit (Roche).

The results of the experiment are shown in Table 12 below.

**[Table 12]**

| Strain | D-lactic acid (mM) | L-lactic acid (mM) |
|---|---|---|
| W. cibaria CHK903 | 10.73 | 0.63 |
| W. cibaria SGW054 | 8.27 | 0.84 |
| W. cibaria SPW2014 | 8.77 | 0.31 |

As shown in Table 12 above, all of the *Weissella cibaria* strains were found to produce D-lactic acid at a higher concentration than L-lactic acid. However, lactic acid bacteria are classified into species that produce L-lactic acid, D-lactic acid, or both L-lactic acid and D-lactic acid, depending on the species, and cannot be considered dangerous because they produce D-lactic acid.

Therefore, it was confirmed that the *Weissella cibaria* strain of the present invention produces both L-lactic acid and D-lactic acid, but does not affect the safety evaluation.

### [Example 15]

### Safety evaluation 10: Bile salt deconjugation activity

Bile salt emulsifies fat and promotes the action of lipase, helping to deconjugate fatty acids. Bile salt hydrolase present in probiotic strains protects probiotic strains from bile salts and increases intestinal colonization, but also deconjugates bile salts, eliminating the emulsification function of bile salts, making fatty acid deconjugation difficult. In addition, in the case of deconjugated bile salts, toxic secondary metabolites may be produced through metabolism by other intestinal microorganisms.

For the *Weissella cibaria* strains shown in Table 2 above, the following experiment was performed to confirm whether they had the bile salt deconjugation activity.

The colonies of lactic acid bacteria were taken, streaked on MRS medium supplemented with 0.5% taurodeoxycholic acid (TDCA; bile acid, Sigma), and then cultured for 2 days at 37 °C under anaerobic conditions. In the case of strains having bile salt hydrolase activity, an opaque white precipitate was formed around the colonies.

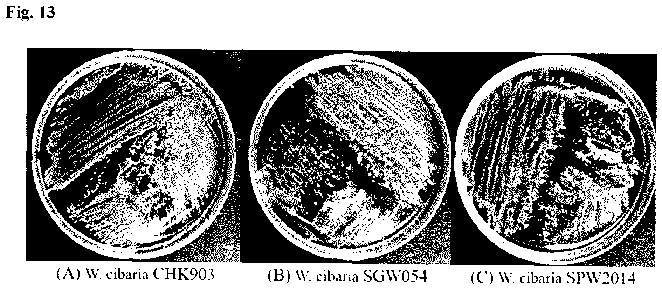

The results of the experiment are shown in Fig. 13. As shown in Fig. 13, it was found that none of the *Weissella cibaria* strains formed opaque white precipitates in the colonies.

Therefore, it was confirmed that the *Weissella cibaria* strain of the present invention does not have the bile salt deconjugation activity and is safe.

### [Example 16]

### Safety evaluation 11: biogenic amine production

Biogenic amines are nitrogen compounds mainly produced by decarboxylation of amino acids, amination of aldehydes and ketones, and transamination reactions. Biogenic amines act directly or indirectly as neurotransmitters in the body and also affect the cardiovascular system, such as blood pressure regulation and blood flow. Therefore, the consumption of foods containing biogenic amines can cause various pharmacological phenomena. In terms of food safety, investigating the distribution and content of biogenic amines is very important. However, in Korea, there are no standards or specifications for biogenic amines produced by probiotic strains.

For the *Weissella cibaria* strains shown in Table 2 above, the following experiment was performed to confirm whether they produced biogenic amines.

Lactic acid bacteria were inoculated into MRS broth supplemented with arginine, ornithine, histidine, tyrosine, tryptophan, lysine, and phenylalanine, which are precursors of biogenic amines, at a concentration of 400 ppm, respectively, and then cultured at 37 °C for 20 hours. After culturing, 500 µL of saturated sodium carbonate solution was added to 1 mL of culture supernatant and mixed, then 800 µL of 1% dansyl chloride acetone solution was added and mixed, and then the mixture was capped and derivatized at 45 °C for 1 hour. 500 µL of 10% proline solution and 5 mL of diethylether were added thereto and mixed thoroughly with a vortex mixer for 10 minutes, and the supernatant was taken and dried by purging with nitrogen gas. After adding 1 mL of acetonitrile to the dried material and dissolving it, the dissolved liquid was filtered through a 0.22 µm membrane filter and used as a test solution for analysis by HPLC. The derivatized biogenic amines were quantitatively analyzed using the DIONEX UltiMate 3000 HPLC system (Thermo Scientific, USA).

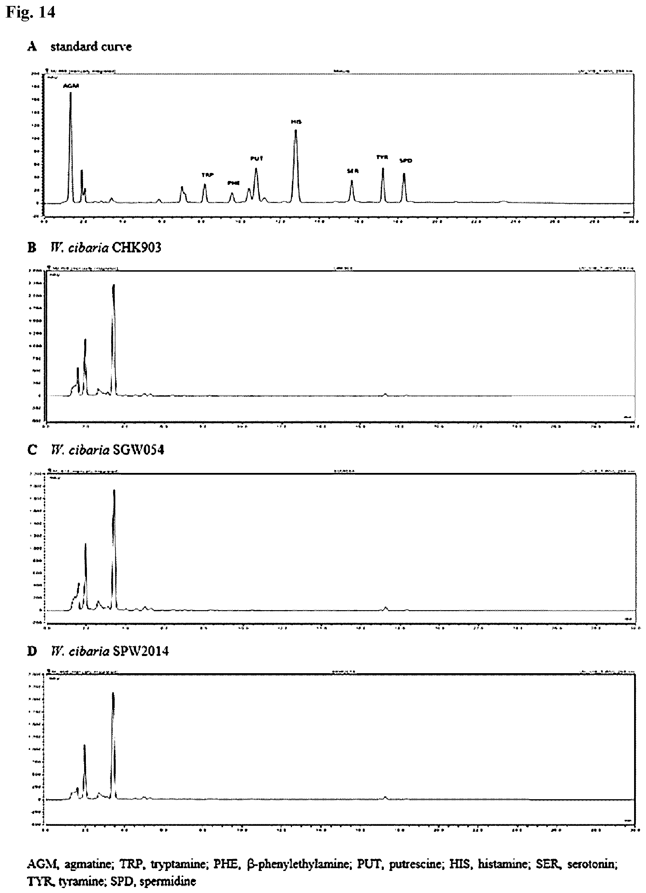

The results of the experiment are shown in Fig. 14. As shown in Fig. 14, in all of the *Weissella cibaria* strains, agmatine, histamine, β-phenylethylamine, putrescine, serotonin, spermidine, tryptamine, and tyramine were not detected.

Therefore, it was confirmed that the *Weissella cibaria* strain of the present invention does not produce biogenic amines and is safe.

Examples 6 to 16 above were carried out in accordance with the Ministry of Food and Drug Safety's 'Guide for Safety Evaluation of Probiotics for Functional Health Functional Food Raw Materials' (June 2021). Ultimately, it can be seen that the *Weissella cibaria* strain of the present invention satisfies probiotic safety standards approved by the Ministry of Food and Drug Safety.

## Claims

1. A pharmaceutical composition for preventing or treating a neurodegenerative disease, comprising a *Weissella cibaria* strain.

2. The pharmaceutical composition for preventing or treating a neurodegenerative disease according to claim 1, wherein the *Weissella cibaria* strain is one or more selected from the group consisting of *Weissella cibaria* CHK903, *Weissella cibaria* SGW054, *Weissella cibaria* SPW2014, *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, and *Weissella cibaria* P26.

3. The pharmaceutical composition for preventing or treating a neurodegenerative disease according to claim 1, wherein the *Weissella cibaria* strain is one or more selected from the group consisting of *Weissella cibaria* CHK903, *Weissella cibaria* SGW054, and *Weissella cibaria* SPW2014.

4. The pharmaceutical composition for preventing or treating a neurodegenerative disease according to claim 1, wherein the pharmaceutical composition inhibits the growth of *Proteus mirabilis.*

5. The pharmaceutical composition for preventing or treating a neurodegenerative disease according to claim 1, wherein the pharmaceutical composition has a protective effect against neurotoxicity-induced apoptosis of neurons.

6. The pharmaceutical composition for preventing or treating a neurodegenerative disease according to claim 1, wherein the pharmaceutical composition has an inhibitory effect on aggregation of α-synuclein.

7. The pharmaceutical composition for preventing or treating a neurodegenerative disease according to claim 1, wherein the pharmaceutical composition has safety by not exhibiting any one or more activities from the group consisting of antibiotic resistance, antibiotic resistance gene, toxicity gene, hemolytic activity, mucin degradation ability, gelatin liquefaction ability, urease activity, indole production ability, beta-glucuronidase (β-glucuronidase) activity, cytotoxicity, bile salt deconjugation activity, and biogenic amine production ability.

8. The pharmaceutical composition for preventing or treating a neurodegenerative disease according to claim 1, wherein the neurodegenerative disease is one or more selected from the group consisting of Parkinson's disease, Alzheimer's disease, Pick's disease, Huntington's disease, Lou Gehrig's disease, prion disease, Lewy body dementia, multiple system atrophy, progressive supranuclear palsy, Friedreich's ataxia, temporal lobe epilepsy, and stroke.

9. The pharmaceutical composition for preventing or treating a neurodegenerative disease according to claim 1, wherein the neurodegenerative disease is Parkinson's disease.

10. A food composition for preventing or ameliorating a neurodegenerative disease, comprising a *Weissella cibaria* strain.

11. The food composition for preventing or ameliorating a neurodegenerative disease according to claim 10, wherein the *Weissella cibaria* strain is one or more selected from the group consisting of *Weissella cibaria* CHK903, *Weissella cibaria* SGW054, *Weissella cibaria* SPW2014, *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, and *Weissella cibaria* P26.

12. The food composition for preventing or ameliorating a neurodegenerative disease according to claim 10, wherein the *Weissella cibaria* strain is one or more selected from the group consisting of *Weissella cibaria* CHK903, *Weissella cibaria* SGW054, and *Weissella cibaria* SPW2014.

13. An animal feed composition for preventing or ameliorating a neurodegenerative disease, comprising a *Weissella cibaria* strain.

14. The animal feed composition for preventing or ameliorating a neurodegenerative disease according to claim 13, wherein the *Weissella cibaria* strain is one or more selected from the group consisting of *Weissella cibaria* CHK903, *Weissella cibaria* SGW054, *Weissella cibaria* SPW2014, *Weissella cibaria* 200022, *Weissella cibaria* SFL001, *Weissella cibaria* FCK913, *Weissella cibaria* JJW001, *Weissella cibaria* SLW6932, *Weissella cibaria* P8, *Weissella cibaria* P9, and *Weissella cibaria* P26.

15. The animal feed composition for preventing or ameliorating a neurodegenerative disease according to claim 13, wherein the *Weissella cibaria* strain is one or more selected from the group consisting of *Weissella cibaria* CHK903, *Weissella cibaria* SGW054, and *Weissella cibaria* SPW2014.

16. A *Weissella cibaria* CHK903 strain (accession number: KCCM13224P).

17. A *Weissella cibaria* SGW054 strain (accession number: KCCM13225P).

18. A *Weissella cibaria* SPW2014 strain (accession number: KCCM13226P).
